# EUROPÄISCHE PATENTSCHRIFT

(11) **EP 3 607 693 B1**
(45) Veröffentlichungstag und Bekanntmachung des Hinweises auf die Patenterteilung: **04.11.2020**
(21) Anmeldenummer: 18717287.9
(22) Anmeldetag: 04.04.2018
(51) Int. Cl.: H04L 7/033, G11B 20/10, G02B 21/00

(54) **VERFAHREN ZUM BILDEN EINES DIGITALWERTS AUS EINEM TAKTSIGNAL UND EINEM DATENSIGNAL**
METHOD FOR FORMING A DIGITAL VALUE FROM A CLOCK SIGNAL AND FROM A DATA SIGNAL
PROCÉDÉ D'ÉTABLISSEMENT D'UNE VALEUR NUMÉRIQUE À PARTIR D'UN SIGNAL D'HORLOGE ET D'UN SIGNAL DE DONNÉES

(30) Priorität: 07.04.2017 DE 102017107560
(43) Veröffentlichungstag der Anmeldung: 12.02.2020
(73) Patentinhaber: Leica Microsystems CMS GmbH, 35578 Wetzlar (DE)
(72) Erfinder: WIDZGOWSKI, Bernd, 69221 Dossenheim (DE)
(74) Vertreter: m patent group
(86) Internationale Anmeldenummer: PCT/EP2018/058523
(87) Internationale Veröffentlichungsnummer: WO 2018/185121

(56) Entgegenhaltungen:
- WO-A1-2016/040884
- M. Kubicek ET AL: "Blind Oversampling Data Recovery with Low Hardware Complexity", Radioengineering, 1. April 2010 (2010-04-01), Seiten 74-78, XP055488892, Gefunden im Internet: URL:http://www.radioeng.cz/fulltexts/2010/ 10_01_074_078.pdf [gefunden am 2018-06-28]
- MING-TA HSIEH ET AL: "Architectures for multi-gigabit wire-linked clock and data recovery", IEEE CIRCUITS AND SYSTEMS MAGAZINE, IEEE SERVICE CENTER, NEW YORK, NY, US, Bd. 8, Nr. 4, 1. Oktober 2008 (2008-10-01) , Seiten 45-57, XP011235829, ISSN: 1531-636X, DOI: 10.1109/MCAS.2008.930152
- AHMED S I ET AL: "Overview of oversampling clock and data recovery circuits", ELECTRICAL AND COMPUTER ENGINEERING, 2005. CANADIAN CONFERENCE ON SASKATOON, SK, CANADA MAY 1-4, 2005, PISCATAWAY, NJ, USA,IEEE, PISCATAWAY, NJ, USA, 1. Mai 2005 (2005-05-01), Seiten 1876-1881, XP010869208, DOI: 10.1109/CCECE.2005.1557348 ISBN: 978-0-7803-8885-7
- Marc Defossez: "An Interface for Texas Instruments Analog-to-Digital Converters with Serial LVDS Outputs", , 7. April 2008 (2008-04-07), XP055488504, Gefunden im Internet: URL:http://xilinx.com/bvdocs/appnotes/xapp 866.pdf [gefunden am 2018-06-27] in der Anmeldung erwähnt

## Beschreibung

Die vorliegende Erfindung betrifft ein Verfahren zum Bilden eines Digitalwerts aus einem Taktsignal und einem digitalen Datensignal sowie eine Recheneinheit zu dessen Durchführung.

### Stand der Technik

In der Mikroskopie, beispielsweise der Konfokalmikroskopie, kommen zahlreiche Sensoren zum Einsatz, deren analoge Ausgangssignale für die Weiterverarbeitung digitalisiert werden müssen. Hierfür können verbreitete Analog-Digital-Umsetzer (ADU bzw. ADC) bzw. Analog-Digital-Wandler eingesetzt werden, die beispielsweise als fertige Bausteine erhältlich sind. Um insbesondere die Verkabelung einfach zu gestalten, werden häufig Analog-Digital-Umsetzer mit serieller Ausgabe eingesetzt, bei denen die Digitalwerte mittels weniger Leitungen (beispielsweise Bitclock, Daten und Frame) als Bitstrom ausgegeben werden. Die Weiterverarbeitung des Bitstroms kann in herkömmlichen integrierten Schaltkreisen (IC) bzw. FPGA (Field Programmable Gate Array) erfolgen, wobei die Methoden hierzu weithin bekannt und in üblichen Bausteinen, wie beispielsweise den Virtex- oder Kintex-FPGAs von Xilinx oder entsprechenden, bereits vorbereitet sind.

Jedoch kann es beim gleichzeitigen Auswerten von zahlreichen Bitströmen von unterschiedlichen ADUs, wie es insbesondere bei der Konfokalmikroskopie mit ihren zahlreichen Photosensoren anzutreffen ist, insbesondere hinsichtlich des Timings zu Problemen kommen. Zum einen müssen bei den bekannten Auswertemethoden insbesondere das Taktsignal (Bit clock) und das Datensignal (Data) wieder synchronisiert werden, da es durch die Signalverarbeitung zunächst zu einer Phasenverschiebung kommt (siehe z.B. "Bit-Clock Alignment" in Xilinx, Application Note: Virtex-4 and Virtex-5 FPGAs, XAPP866 (v3.0) April 7, 2008). Zum anderen verschärft sich dieses Problem, wenn mehrere ADUs beteiligt sind, die sich in unterschiedlicher Entfernung vom Baustein befinden.

Ein blindes Datenabtastverfahren mit mehreren gegenseitig phasenverschobenen Taktsignalen ist in "Blind Oversampling Data Recovery with Low Hardware Complexity", Michal Kubíček, Zdeněk Kolka, Radioengineering, Vol. 19, No. 1, April 2010, vorgestellt.

### Offenbarung der Erfindung

Erfindungsgemäß werden ein Verfahren zum Bilden eines Digitalwerts aus einem Taktsignal und einem digitalen Datensignal sowie eine Recheneinheit zu dessen Durchführung mit den Merkmalen der unabhängigen Patentansprüche vorgeschlagen. Vorteilhafte Ausgestaltungen sind Gegenstand der Unteransprüche sowie der nachfolgenden Beschreibung.

Die vorliegende Erfindung basiert auf der Idee, die Digitalwertbildung im Auswertebaustein (IC bzw. FPGA) nicht mittels herkömmlicher Methoden, die mit den eingangs genannten Problemen behaftet sind, durchzuführen, sondern sich einer ("einfachen") Signalabtastung zu bedienen, welche ebenfalls in den genannten Bausteinen vorbereitet und mit einer sehr hohen Abtastrate im GHz-Bereich möglich ist. Auf diese Weise werden aus den vom ADU ausgegebenen Signalen (zumindest Taktsignal und Datensignal) Signaldigitalwertefolgen erzeugt und verarbeitet. Da die Abtastung der unterschiedlichen Signale im Wesentlichen gleichzeitig stattfindet, kommt es nicht zu einer Phasenverschiebung zwischen Signalen oder anderen Timingproblemen. Somit wird durch die vorliegende Erfindung eine robuste, genaue und einfach zu implementierende Methode zum Bilden eines Digitalwerts insbesondere aus den Ausgabesignalen eines ADUs mit serieller Ausgabe vorgestellt.

Vorzugsweise werden die Abtastzeitpunkte aus Werteveränderungen in der Taktsignaldigitalwertefolge bestimmt. Die Bestimmung von Abtastzeitpunkten, zu denen das Datensignal die für die Weiterverarbeitung benötigten Werte aufweist, kann nämlich sehr einfach durch Analyse des Taktsignals erfolgen. Übliche Abtastzeitpunkte liegen an den Flanken des Taktsignals oder (vorzugsweise mittig) zwischen den Flanken. Die konkrete Beziehung zwischen geeigneten Abtastzeitpunkten und Flanken geht aus dem Datenblatt des verwendeten ADUs hervor. Somit sind, im Unterschied zum Stand der Technik, keine aufwendigen Maßnahmen, wie zum Beispiel das Vorsehen von Zustandsautomaten und davon angesteuerten Verzögerungsgliedern, notwendig.

Zweckmäßigerweise ist die Taktsignaldigitalwertefolge eine binäre Wertefolge, d.h. eine aus nur zwei Werten (üblicherweise "0" und "1") bestehende Wertefolge. Dadurch kann der Abtast- und Rechenaufwand reduziert werden, ohne dass die Auswertung negativ beeinflusst würde.

Gemäß einer bevorzugten Ausführungsform werden das Taktsignal und/oder das digitale Datensignal überabgetastet. Vorzugsweise ist die Abtastfrequenz das Achtfache oder 16-fache der Frequenz des Taktsignals. Bei einer Überabtastung des Taktsignals können insbesondere dessen Flanken, die üblicherweise maßgeblich für den Abtastzeitpunkt des Datensignals sind, zeitlich besonders genau bestimmt werden. Bei einer Überabtastung des Datensignals, vorzugsweise mit derselben Abtastrate wie das Taktsignal, stehen ausreichend viele und - in Abhängigkeit von der Abtastrate - auch genau oder nahezu genau an den Abtastzeitpunkten abgetastete Datensignaldigitalwerte zur Verfügung, so dass diese zur Bildung des Digitalwerts auch einfach ausgewählt werden können. Grundsätzlich ist es jedoch ausreichend, wenn das Datensignal nur an den Abtastzeitpunkten abgetastet wird, soweit diese früh genug bekannt sind.

Vorzugsweise ist die Datensignaldigitalwertefolge eine binäre Wertefolge. Da es sich bei einem Datensignal, welches von einem herkömmlichen ADU mit serieller Ausgabe stammt, um einen Binärsignal handelt, ist es auch ausreichend, die Datensignaldigitalwertefolge als binäre Wertefolge zu realisieren. Dadurch kann der Abtast- und Rechenaufwand reduziert werden, ohne dass die Auswertung negativ beeinflusst würde.

Eine besonders bevorzugte Möglichkeit zur Bildung des Digitalwerts umfasst, dass die Datensignaldigitalwerte die Bits des Digitalwerts bilden. Somit sind zur Bildung des Digitalwerts lediglich einfache Operationen nötig.

Da herkömmliche ADUs üblicherweise auch ein Rahmensignal (Frame Clock) ausgeben, wird dies gemäß einer Weiterbildung der vorliegenden Erfindung vorzugsweise ebenfalls abgetastet. Die sich daraus ergebende Rahmensignaldigitalwertefolge kann dann vorteilhafterweise zusätzlich dazu verwendet werden, den Digitalwert zu bilden. Beispielsweise kann das Rahmensignal anzeigen, welche Bits aus dem Datensignal zu demselben Digitalwert gehören. Beispielsweise entspricht jede Periode des Rahmensignals einem Digitalwert.

Gewünschtenfalls können Signale und/oder Signaldigitalwertefolge gefiltert werden, um Störungen zu entfernen. Insbesondere können Tiefpassfilter eingesetzt werden, um Rauschen u.ä. auszufiltern.

Eine erfindungsgemäße Recheneinheit, z.B. eine integrierte Schaltung (IC) oder ein FPGA, insbesondere in einem Steuergerät eines Mikroskops, ist dazu eingerichtet, ein erfindungsgemäßes Verfahren durchzuführen.

Die Erfindung kann vorteilhaft in der Mikroskopie, insbesondere KonfokalMikroskopie, eingesetzt werden, da dort zahlreiche Signale, wie z.B. von Photomultipliern, Arrays von Avalanche-Photodioden (Silicon Photomultiplier, SiPM), Photodioden, dem Analogausgang eines Lock-In-Verstärkers oder einem vom Anwender aus seiner Messapparatur generierten analogen Signal, welches synchron zum Bild aufgezeichnet werden soll, möglichst gleichzeitig erfasst werden sollen, da in den daraus generierten Bildern an einer Pixelposition der Zustand der Sensoren zum gleichen Zeitpunkt dargestellt werden muss. Werden mehrere Bilder hintereinander aufgenommen, dann spiegelt die Bilderfolge zeitliche Veränderung der Probe wider. Die zeitliche Korrelation ist also bei allen zeitvarianten Proben notwendig.

Weitere Vorteile und Ausgestaltungen der Erfindung ergeben sich aus der Beschreibung und der beiliegenden Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand eines Ausführungsbeispiels in der Zeichnung schematisch dargestellt und wird im Folgenden unter Bezugnahme auf die Zeichnung beschrieben.

### Figurenbeschreibung

Figur 1 zeigt schematisch typische Signale, wie sie von einem ADU mit serieller Ausgabe ausgegeben werden.
Figur 2 zeigt schematisch die Abtastung der Signale aus Figur 1 gemäß einer bevorzugten Ausführungsform der Erfindung.
Figur 3 zeigt schematisch eine bevorzugte Ausführungsform einer erfindungsgemäßen Recheneinheit.

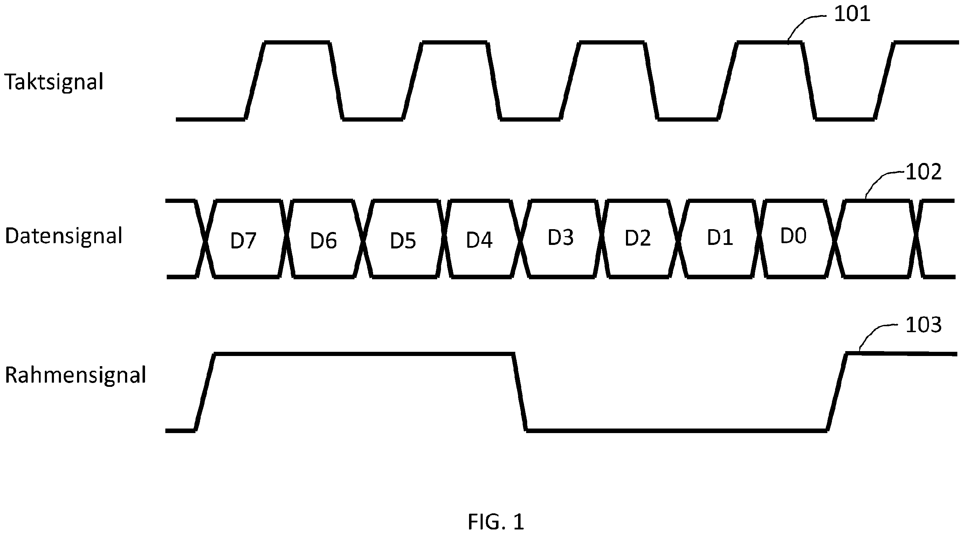

In Figur 1 sind schematisch drei typische Signale, wie sie von einem ADU mit serieller Ausgabe ausgegeben werden, dargestellt. Insbesondere sind ein mit 101 bezeichnetes Taktsignal (bit_clock), ein mit 102 bezeichnetes Datensignal (data) und ein mit 103 bezeichnetes Rahmensignal (frame_clock) dargestellt.

Das Taktsignal 101 kennzeichnet den Grundtakt der Signale und dient insbesondere zur Identifizierung der Bits im Datensignal 102. Bei der dargestellten Ausführungsform kennzeichnet insbesondere jede steigende und jede fallende Flanke im Taktsignal ein Bit im Datensignal. Es handelt sich somit um ein sogenanntes DDR-Signal (double data rate). Das Rahmensignal 103 dient zur Kennzeichnung derjenigen Bits, die zum selben Digitalwert gehören. Im vorliegenden Beispiel sind acht beispielhafte Bits eines Digitalwerts (Byte) im Datensignal mit D0 bis D7 gekennzeichnet. Grundsätzlich können jedoch auch mehr als 8 Bits zu einem Digitalwert gehören, verbreitet sind z.B. 10, 12 oder 16. Abhängig vom konkreten Wert (z.B. "0" oder "1") ist der Signalpegel bei jedem Bit "High" oder "Low".

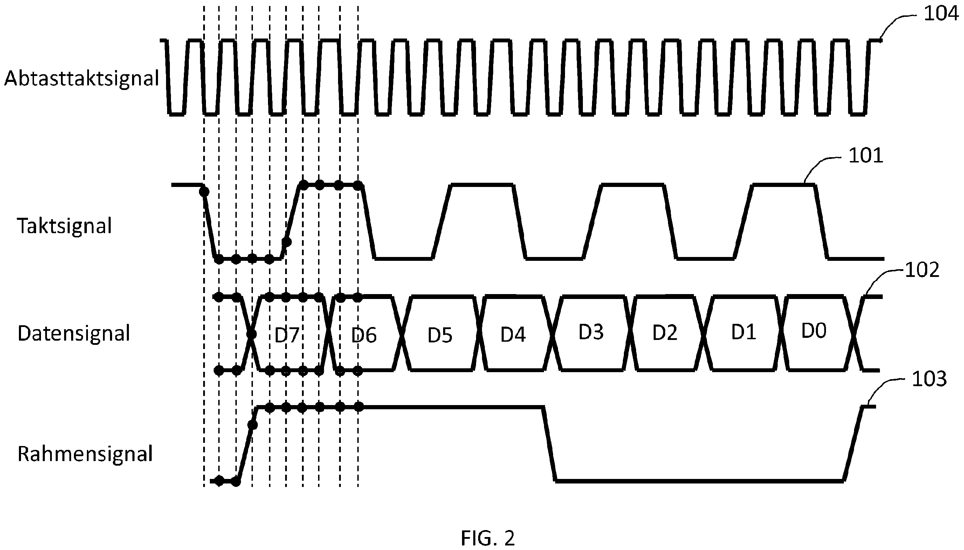

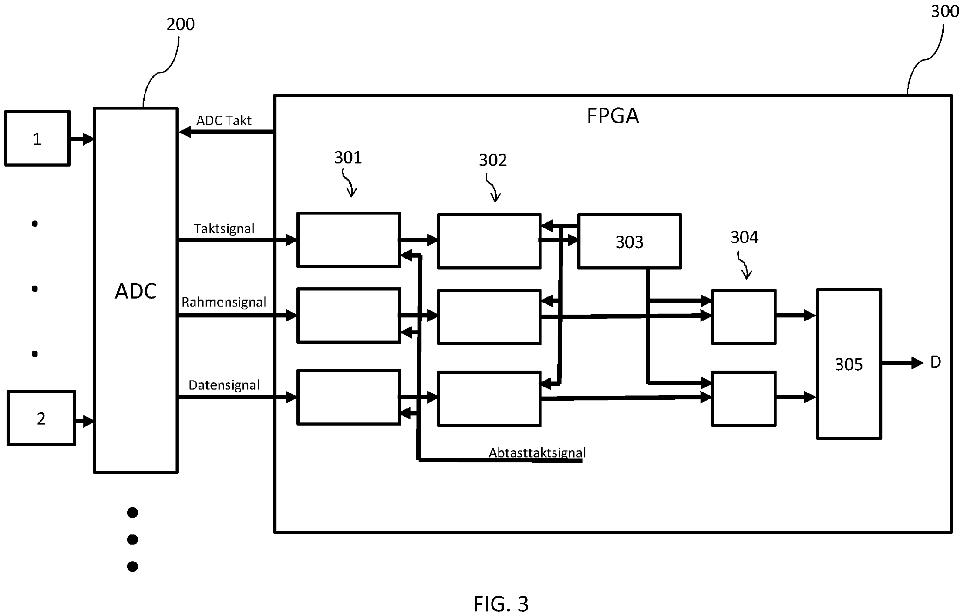

Unter Bezugnahme auf die Figuren 2 und 3 wird im Folgenden eine bevorzugte Ausführungsform der Erfindung beschrieben, wobei Figur 2 schematisch die Abtastung der Signale aus Figur 1 und Figur 3 schematisch eine bevorzugte Ausführungsform einer erfindungsgemäßen Recheneinheit zeigen.

In Figur 3 ist eine beispielhafte Anordnung aus einer Recheneinheit 300 gemäß einer bevorzugten Ausführungsform der Erfindung, einem oder mehreren mit dieser verbundenen Analog-Digital-Umsetzern 200 (ADC) mit serieller Ausgabe und jeweils mit einem von dem einen oder den mehreren ADCs 200 verbundenen Sensoren 1,2,... dargestellt. Eine solche Anordnung kann insbesondere in Messgeräten vorteilhaft zum Einsatz kommen, die zahlreiche analoge Signale gleichzeitig erfassen müssen, wie insbesondere Konfokalmikroskope.

Bei den Sensoren 1, 2, ... kann es sich um beliebige Sensoren handeln, bei Konfokalmikroskopen beispielsweise um Fotodetektoren. Bei den ADCs 200 kann es sich um herkömmliche insbesondere mehrkanalige Analog-Digital-Umsetzer mit serieller Ausgabe handeln. Bei der Recheneinheit 200 handelt es sich beispielsweise um ein herkömmliches FPGA (Field Programmable Gate Array).

Die vom ADC 200 gelieferten Signale werden dem FPGA 300 zugeführt und, wie in Figur 2 dargestellt, abgetastet, wozu Abtastglieder 301 dienen, deren Abtastrate insbesondere einstellbar ist.

In Figur 2 ist dargestellt, dass das Datensignal 101, das Taktsignal 101 und das Rahmensignal 103 abgetastet werden, um entsprechende Signaldigitalwertefolgen zu erhalten. Beispielsweise wird eine Abtastrate durch ein Abtasttaktsignal 104 (sample_clock) vorgegeben. Beispielsweise erfolgt die Abtastung sowohl bei steigender als auch bei fallender Flanke des Abtasttaktsignals 104, wobei die sich ergebenden Messwerte in den Signalen 101-103 durch ausgefüllte bzw. offene Kreise angedeutet sind. Es ist erkennbar, dass in dem dargestellten Beispiel die Signale 101-103 überabgetastet werden, wobei die Abtastfrequenz etwa das 4,5-bis 5-fache der Frequenz des Taktsignals 101 ist.

Zur Vereinfachung der Auswertung und Weiterverarbeitung werden als Signaldigitalwertefolgen binäre Wertefolgen verwendet, welche nur aus den Werten "0" und "1" bestehen. Für diesen Zweck eignen sich als Abtastglieder 301 insbesondere sog. Deserialisierer. Dieser besteht beispielsweise aus einer Anordnung von FlipFlops, die auf bestimmte Art verschaltet sind. An den zugehörigen Eingängen des FPGA befinden sich vorteilhafterweise nur Schwellwertschalter, um zwischen den Spannungspegeln, die die logischen Signale repräsentieren, zu unterscheiden.

Im gezeigten Beispiel besteht die Taktsignaldigitalwertefolge somit aus 1000001111..., die Datensignaldigitalwertefolge z.B. aus 1111111111... (ausgefüllte Kreise) und die Rahmensignaldigitalwertefolge aus 0011111111...., mithin also:

| Stelle | 1 | 2 | 3 | 4 | 5 | 6 | 7 | 8 | 9 | 10 | |
|---|---|---|---|---|---|---|---|---|---|---|---|
| bit_clock | 1 | 0 | 0 | 0 | 0 | 0 | 1 | 1 | 1 | 1 | ... |
| data | 1 | 1 | 1 | 1 | 1 | 1 | 1 | 1 | 1 | 1 | ... |
| frame_clock | 0 | 0 | 1 | 1 | 1 | 1 | 1 | 1 | 1 | 1 | .... |

Die Signaldigitalwertefolgen werden Speichergliedern 302, beispielsweise sog. Barrel-Shifter, zugeführt. Die Speicherglieder 302 dienen zum Zwischenspeichern und gezielten Weitergeben von Teilen der Signaldigitalwertefolgen, um diese weiterverarbeiten zu können. Die Breite jedes der Speicherglieder 302 muss so bemessen sein, dass mindestens eine Periode des Taktsignals 101 gespeichert ist, hier also mindestens 9-10 Bits. Vorteilhafterweise ist jedes der Speicherglieder 302 so bemessen, dass zwei Perioden dieses Taktsignals hineinpassen. Dies vereinfacht das Auffinden von Pegelwechseln.

Jede zwischengespeicherte Teil-Taktsignaldigitalwertefolge wird einem Auswerteglied 303 zugeführt, welches die Teil-Taktsignaldigitalwertefolge auf Werteveränderungen untersucht. Im obigen Beispiel ist eine Werteveränderung von 1 auf 0 von der ersten zur zweiten Stelle und von 0 auf 1 von der sechsten zur siebten Stelle detektierbar. Diese Werteveränderungen werden als Flanken im Taktsignal 101 verstanden. In Kenntnis des verwendeten ADCs kann daraus als geeigneter Abtastzeitpunkt für das Datensignal 102 beispielsweise die vierte Stelle ermittelt werden. Diese Information wird an Extrahierglieder 304 übermittelt, die die betreffenden Bits aus der Teil-Datensignaldigitalwertefolge und Teil-Rahmensignaldigitalwertefolge extrahieren und einem Digitalwertbildungsglied 305 zuführen.

Sind mehrere unterschiedliche ADCs 200 mit dem FPGA 300 verbunden, sind üblicherweise auch die jeweiligen Entfernungen zwischen ADC und FPGA unterschiedlich, so dass die Signale der unterschiedlichen ADCs 200 mit unterschiedlichen Phasenbeziehungen vorliegen. In einem solchen Fall können die Speicherglieder 302 oder spezielle Verzögerungsglieder (nicht gezeigt) dazu dienen, die Phasen zu synchronisieren, d.h. früher eintreffende Signale zu verzögern, bis sie mit später eintreffenden möglichst phasengleich sind.

Weiterhin veranlasst das Auswerteglied 303 die Weitergabe einer neuen Teil-Taktsignaldigitalwertefolge, Teil-Datensignaldigitalwertefolge und Teil-Rahmensignaldigitalwertefolge aus den Speichergliedern 302 in Abhängigkeit vom Fortgang der Auswertung.

Nach einer ausreichend großen Anzahl von Schritten liegen dann in dem Digitalwertbildungsglied 305 alle zur Bildung des Digitalwerts D nötigen Informationen (hier Bits) vor und der Digitalwert D wird gebildet.

## Patentansprüche

1. Verfahren zum Bilden eines Digitalwerts (D) aus einem Taktsignal (101) und einem digitalen Datensignal (102),
wobei das Taktsignal (101) abgetastet wird, um eine Taktsignaldigitalwertefolge zu erhalten,
wobei das digitale Datensignal (102) abgetastet wird, um eine Datensignaldigitalwertefolge zu erhalten,
wobei aus der Taktsignaldigitalwertefolge Abtastzeitpunkte ermittelt werden, zu welchen aus der Datensignaldigitalwertefolge Datensignaldigitalwerte extrahiert werden,
wobei aus den Datensignaldigitalwerten der Digitalwert (D) gebildet wird.

2. Verfahren nach Anspruch 1, wobei die Abtastzeitpunkte aus Werteveränderungen in der Taktsignaldigitalwertefolge bestimmt werden.

3. Verfahren nach Anspruch 1 oder 2, wobei die Taktsignaldigitalwertefolge eine binäre Wertefolge ist.

4. Verfahren nach einem der vorstehenden Ansprüche, wobei das Taktsignal (101) überabgetastet wird.

5. Verfahren nach einem der vorstehenden Ansprüche, wobei das digitale Datensignal (102) überabgetastet wird oder nur zu den Abtastzeitpunkten abgetastet wird, um die Datensignaldigitalwertefolge zu erhalten.

6. Verfahren nach einem der vorstehenden Ansprüche, wobei die Datensignaldigitalwertefolge eine binäre Wertefolge ist.

7. Verfahren nach Anspruch 6, wobei aus den Datensignaldigitalwerten der Digitalwert gebildet wird, indem die Datensignaldigitalwerte die Bits des Digitalwerts bilden.

8. Verfahren nach einem der vorstehenden Ansprüche, wobei ein Rahmensignal (103) abgetastet wird, um eine Rahmensignaldigitalwertefolge zu erhalten, wobei der Digitalwert (D) zusätzlich unter Verwendung der Rahmensignaldigitalwertefolge gebildet wird.

9. Verfahren nach einem der vorstehenden Ansprüche, wobei das Taktsignal (101) und das digitalen Datensignal (102) von einem Analog-Digital-Umsetzer mit (200) serieller Ausgabe ausgegeben werden.

10. Recheneinheit (300), die dazu eingerichtet ist, ein Verfahren nach einem der vorstehenden Ansprüche durchzuführen.

11. Recheneinheit (300) nach Anspruch 10, die als integrierte Schaltung, insbesondere als FPGA, ausgebildet ist.

12. Mikroskop mit einer Recheneinheit (300) nach Anspruch 10 oder 11.

## Claims

1. A method for forming a digital value (D) from a clock signal (101) and a digital data signal (102),
wherein the clock signal (101) is sampled to obtain a clock signal digital value sequence,
wherein the digital data signal (102) is sampled to obtain a data signal digital value sequence,
wherein sampling times are determined from the clock signal digital value sequence, at which sampling times data signal digital values are extracted from the data signal digital value sequence,
wherein the digital value (D) is formed from the data signal digital values.

2. The method according to claim 1, wherein the sampling times are determined from value changes in the clock signal digital value sequence.

3. The method according to claim 1 or 2, wherein the clock signal digital value sequence is a binary value sequence.

4. The method according to any one of the preceding claims, wherein the clock signal (101) is oversampled.

5. The method according to any one of the prededing claims, wherein the digital data signal (102) is oversampled or is sampled only at the sampling times to obtain the data signal digital value sequence.

6. The method according to any one of the preceding claims, wherein the data signal digital value sequence is a binary value sequence.

7. The method according to claim 6, wherein the digital value is formed from the data signal digital values by the data signal digital values forming the bits of the digital value.

8. The method according to any one of the preceding claims, wherein a frame signal (103) is sampled to obtain a frame signal digital value sequence, wherein the digital value (D) is additionally formed using the frame signal digital value sequence.

9. The method according to any of the preceding claims, wherein the clock signal (101) and the digital data signal (102) are output from an analog-to-digital converter with (200) serial output.

10. A computing unit (300) adapted to perform the method according to one of the preceding claims.

11. The computing unit (300) according to claim 10, which is designed as an integrated circuit, in particular as an FPGA.

12. A microscope with a computing unit (300) according to claim 10 or 11.

## Revendications

1. Un procédé de formation d'une valeur numérique (D) à partir d'un signal d'horloge (101) et d'un signal de données numérique (102),
dans laquelle le signal d'horloge (101) est échantillonné pour obtenir une séquence de valeurs numériques du signal d'horloge,
dans laquelle le signal de données numériques (102) est échantillonné pour obtenir une séquence de valeurs du signal de données numérique,
où les temps d'échantillonnage sont déterminés à partir de la séquence de valeurs numériques du signal d'horloge, et où les temps d'échantillonnage des valeurs du signal de données numérique sont extraits de la séquence de valeurs signal de données numérique,
où la valeur numérique (D) est formée à partir des valeurs du signal de données numérique.

2. Le procédé selon la revendication 1, dans lequel les temps d'échantillonnage sont déterminés à partir des changements de valeur dans la séquence de valeurs numériques du signal d'horloge.

3. Le procédé selon la revendication 1 ou 2, dans lequel la séquence de valeurs numériques du signal d'horloge est une séquence de valeurs binaires.

4. Le procédé selon l'une des revendications ci-dessus, dans lequel le signal d'horloge (101) est suréchantillonné.

5. Le procédé selon l'une des revendications ci-dessus, dans lequel le signal de données numériques (102) est suréchantillonné ou n'est échantillonné qu'aux temps d'échantillonnage pour obtenir la séquence de valeurs du signal de données numérique.

6. Le procédé selon l'une des revendications ci-dessus, dans lequel la séquence de valeurs numériques du signal de données est une séquence de valeurs binaires.

7. Le procédé selon la revendication 6, dans lequel la valeur numérique est formée à partir des valeurs numériques du signal de données par les valeurs du signal de données numérique formant les bits de la valeur numérique.

8. Le procédé selon l'une des revendications ci-dessus, dans lequel un signal de trame (103) est échantillonné pour obtenir une séquence de valeurs de signal de trame numérique, dans lequel la valeur numérique (D) est en outre formée en utilisant la séquence de valeurs numériques de signal de trame.

9. Le procédé selon l'une des revendications précédentes, dans lequel le signal d'horloge (101) et le signal de données numériques (102) sont émis par un convertisseur analogique-numérique avec une sortie série (200).

10. Une unité de calcul (300) adaptée pour exécuter un procédé selon l'une des revendications précédentes.

11. L'unité de calcul (300) selon la revendication 10, qui est conçue comme un circuit intégré, en particulier comme un FPGA.

12. Un microscope avec une unité de calcul (300) selon la revendication 10 ou 11.
